# FASCICULE DE BREVET EUROPEEN

(11) **EP 3 551 164 B1**
(45) Date de publication et mention de la délivrance du brevet: **31.03.2021**
(21) Numéro de dépôt: 16822491.3
(22) Date de dépôt: 08.12.2016
(51) Int. Cl.: A61K 9/00, A61K 9/06, A61K 47/44, A61K 31/77

(54) **MÉDICAMENT ORAL COMPRENANT UN LAXATIF OSMOTIQUE INCORPORÉ DANS UNE MATRICE À BASE DE MATIÈRES GRASSES VÉGÉTALES**
ORALES ARZNEIMITTEL MIT EINEM OSMOTISCHEN LAXATIVUM IN EINER MATRIX AUF DER BASIS VON PFLANZLICHEN FETTEN
ORAL MEDICAMENT COMPRISING AN OSMOTIC LAXATIVE INCORPORATED INTO A MATRIX BASED ON PLANT FATS

(43) Date de publication de la demande: 16.10.2019
(73) Titulaire: Salsarulo Pharma, 92100 Boulogne-Billancourt (FR)
(72) Inventeur: SALSARULO, Gérard, 92100 Boulogne-Billancourt (FR); SALSARULO, Gilles, 92100 Boulogne-Billancourt (FR)
(74) Mandataire: Gevers & Orès
(86) Numéro de dépôt international: PCT/FR2016/053270
(87) Numéro de publication internationale: WO 2018/104591

(56) Documents cités:
- EP-A1- 0 911 038
- WO-A1-2013/044085
- FR-A1- 2 852 843
- MORRIS GORDON ET AL: "Cochrane Review: Osmotic and stimulant laxatives for the management of childhood constipation (Review)", EVIDENCE-BASED CHILD HEALTH: A COCHRANE REVIEW JOURNAL, vol. 8, no. 1, 1 janvier 2013 (2013-01-01) , pages 57-109, XP055273738, GB ISSN: 1557-6272, DOI: 10.1002/ebch.1893 cité dans la demande
- SUPORN TREEPONGKARUNA ET AL: "A randomised, double-blind study of polyethylene glycol 4000 and lactulose in the treatment of constipation in children", BMC PEDIATRICS, BIOMED CENTRAL, LONDON, GB, vol. 14, no. 1, 19 juin 2014 (2014-06-19), page 153, XP021190250, ISSN: 1471-2431, DOI: 10.1186/1471-2431-14-153 cité dans la demande
- ABUT E ET AL: "Administration of olive oil followed by a low volume of polyethylene glycol-electrolyte lavage solution improves patient satisfaction with right-side colonic cleansing over administration of the conventional volume of polyethylene glycol-electrolyte lavage solution for colonoscopy preparation", GASTROINTESTINAL ENDOSCOPY, ELSEVIER, NL, vol. 70, no. 3, 1 septembre 2009 (2009-09-01), pages 515-521, XP026494054, ISSN: 0016-5107, DOI: 10.1016/J.GIE.2009.01.002 [extrait le 2009-06-24]

## Description

La présente invention se rapporte au domaine des préparations pharmaceutiques laxatives à base de macrogol (PEG) ; elle permet la réduction importante des doses quotidiennes de macrogol entrainant de ce fait une réduction, voire une quasi disparition, des effets secondaires découlant des doses quotidiennes élevées de ce type de laxatif osmotique.

La présente invention repose sur l'incorporation du macrogol, aussi désigné PEG dans ce qui suit, dans une matrice hydrophobe à base de matières grasses végétales ayant un point de fusion à la température du corps humain et plus spécifiquement compris entre 36°C et 38°C, et son administration par voie orale (par exemple sous forme de gel ou de pâte).

Les laxatifs osmotiques, tels que le macrogol, sont une classe de laxatifs captant de l'eau conduisant ainsi au gonflement et au ramollissement des selles et permettant de traiter les inconvénients de la constipation.

Le mécanisme d'action de ce type de laxatif nécessite toutefois qu'il produise son effet au niveau du colon (site reconnu de l'action des laxatifs osmotiques), c'est-à-dire qu'il conserve une pression osmotique maximale jusqu'à ce site d'action ; en conséquence, une formulation optimale est telle qu'elle évite le contact du macrogol avec l'humidité du tractus digestif qui diminue la pression osmotique, base de l'efficacité laxative du macrogol, et ne libère le macrogol que dans le colon.

Une solution à ce problème est proposée dans la Demande de Brevet Européen EP 0 911 038 qui décrit une formulation de lactulose, autre laxatif osmotique, dans un véhicule d'enrobage constitué d'un mélange d'hydrocarbures paraffiniques et qui n'est pas végétal ; cette formulation permet de réduire les doses de lactulose habituellement utilisées, comprises entre 10 à 30 g par jour pour un adulte, à 5 à 10 g par jour pour un adulte et ainsi de réduire, voire d'éviter, les effets secondaires indésirables de ce type d'actifs (ballonnements, flatulences, crampes abdominales) dus à des doses élevées de laxatifs osmotiques.

Il existe toutefois un préjugé à l'utilisation médicale d'hydrocarbures paraffiniques en raison de leur origine.

En outre, ces composés paraffiniques ont eux-mêmes un effet lubrifiant sur le transit intestinal et contribuent activement à l'effet laxatif de la composition.

Divers documents décrivent l'utilisation du macrogol :
Par exemple, la Demande Internationale PCT WO 2013/044085 décrit des compositions laxatives solides sous forme de barres, chocolatées par exemple, ou de bonbons ; l'objectif de ce type de formulations est de masquer le gout de principes actifs. Il convient de noter que les formulations exemplifiées dans ce document comprennent des ingrédients hydrophiles (sels, sucralose, poudre de lait présent dans le chocolat blanc) et de l'eau qui ne permettent pas de conserver le caractère anhydre du macrogol jusqu'à son site d'action ; en outre, rien ne permet de penser que le véhicule des formulations proposées a un point de fusion compris entre 36 et 38°C.

D'autres publications, comme la revue clinique « Osmotic and stimulant laxatives for the management of childhood constipation » de Gordon et al. (A COCHRANE REVIEW JOURNAL, vol. 8, n°1, 01/01/2013, p. 57-109) qui compile les données de différents essais cliniques de laxatifs ou encore l'article « A randominsed, double-blind study of polyethylene glycol 4000 and lactulose in the treatment of constipation in children » de Treepongkaruna et al. (BMC PEDIATRICS, BIOMED CENTRAL, LONDON, GB, vol. 14, n°1, 19/06/2014, p. 153) qui décrit une étude comparant l'effet de l'administration quotidienne de 3,3 g de lactulose ou 8 g de PEG pour le traitement de la constipation chez l'enfant, proposent l'administration de doses quotidiennes de macrogol faibles mais il s'agit de doses pédiatriques qui, rapportées au poids du patient à traiter, correspondent à des doses classiques, c'est-à-dire trop élevées et qui induisent des effets secondaires indésirables.

Cependant aucun de ces documents ne propose une galénique de ce laxatif qui soit suffisamment performante pour pouvoir réduire les doses quotidiennes de macrogol administrées.

La Demanderesse s'est ainsi donné pour objectif de développer un médicament laxatif pour le traitement de la constipation permettant de réduire les doses journalières du PEG pour amoindrir les effets secondaires résultant de l'administration de fortes doses de laxatif et a mis au point une nouvelle formulation galénique de PEG à base de composés végétaux et non paraffiniques :
- qui préserve l'intégralité de la pression osmotique du PEG, base de son effet laxatif, jusqu'à son site d'action, le colon ;
- qui est insensible aux valeurs du pH du tractus digestif ;
- qui, de façon étonnante, permet l'utilisation de doses réduites de PEG, comprises entre 5 et 15 g/jour, c'est-à-dire aussi faibles que dans le cas de l'utilisation d'un véhicule paraffinique, et ayant pour conséquence la réduction importante des effets secondaires indésirables (crampes abdominales, ballonnements, flatulences...) ;
- dont le véhicule est principalement d'origine végétale.

La Demanderesse est parvenue à ce résultat , qui se trouve défini dans le jeu de revendications, en utilisant à titre de véhicule un enrobage lipidique anhydre et hydrophobe composé de cires ou beurres d'origine végétale, éventuellement additionnées d'huiles végétales pour obtenir un point de fusion compris entre 36 et 38°C ; de façon surprenante, ce véhicule, qui s'appuie sur une sélection particulière d'excipients classiquement utilisés pour la formulation de médicaments, augmente avantageusement l'efficacité du macrogol et permet l'utilisation de doses quotidiennes réduites de macrogol.

La Demande de Brevet Français FR 2 852 843 décrit des formulations pharmaceutiques consistant en des particules lipidiques solides obtenues par un procédé de préparation particulier de formation de gouttelettes lipidiques dans un gel ; il n'est pas question dans cette Demande de Brevet de chercher à réduire les doses de principe actif. Cette Demande de Brevet a pour objectif la mise au point d'un procédé de fabrication spécifique utilisant des gouttelettes lipidiques et résultant en des formulations permettant le masquage du goût de principes actifs ; cette Demande envisage l'utilisation d'une vaste liste d'excipients gras incluant des excipients non végétaux. En outre, la préparation de ces formes solides nécessite la mise en œuvre d'un procédé plus complexe que celui permettant la préparation de la composition pharmaceutique selon l'invention.

La composition pharmaceutique orale selon l'invention a pour but le maintien de la pression osmotique du macrogol lors de son administration orale d'où la réduction de la posologie journalière de ce laxatif et est constituée de :
- entre 30 et 55% en poids de la composition pharmaceutique de macrogol anhydre ; le macrogol peut être micronisé, dans ce cas, la taille de particules se situe de préférence entre 75 et 150 µm ; et
- entre 45 et 70% en poids de la composition pharmaceutique d'un véhicule constitué d'un enrobage lipidique anhydre et hydrophobe à base de composés gras d'origine végétale ; plus particulièrement, cet enrobage lipidique anhydre et hydrophobe est composé :
   *soit jusqu'à 100% en poids du véhicule lorsqu'il s'agit de beurre de karité et/ou de beurre de cacao ; ou
   *soit de 12 à 25%, de préférence de 15 à 25%, en poids du véhicule d'une cire végétale ; et, selon qu'il s'agit de beurre végétal ou de cire végétale, de 25 à 88%, de préférence de 25 à 85%, en poids du véhicule d'une huile végétale permettant d'ajuster le point de fusion dudit véhicule entre 36 et 38°C
- entre 0 et 5 % en poids de la composition pharmaceutique d'un excipient anhydre choisi parmi des fluidifiants, des agents améliorant l'homogénéité, émulsifiants, arômes et/ou édulcorants à l'exclusion des dérivés cellulosiques.

Par laxatif osmotique et anhydre, on entend du macrogol en poudre qui contient moins de 0,5% d'eau.

Les composés gras d'origine végétale du véhicule lipidique anhydre et hydrophobe sont choisis parmi une sélection spécifique de produits couramment utilisés par l'homme de l'art pour des enrobages hydrophobes, à température de fusion encadrée comme la cire de carnauba, la cire de candelilla, la cire de son de riz, le beurre de karité, le beurre de cacao ou autre cire végétale pharmaceutiquement acceptable.

Afin d'atteindre un point de fusion de 36 à 38°C, certaines cires et certains beurres demandent l'adjonction d'huile végétale anhydre et hydrophobe telle que l'huile de tournesol, l'huile de colza, l'huile de maïs, l'huile de lin ou leur mélange.

Si l'on souhaite améliorer l'homogénéité de la composition selon l'invention, le véhicule lipidique anhydre et hydrophobe peut en outre comprendre certains composés végétaux ou non tels que, par exemple, des glycérides et/ou des glycérostéarates.

Le point de fusion du véhicule lipidique anhydre et hydrophobe peut être classiquement mesuré avec une variabilité de +/- 5% par la mise en œuvre de la technique décrite dans la Pharmacopée Française en vigueur ou par une méthode similaire.

La composition selon l'invention se présente sous forme de pâte ou de gel à administrer oralement.

Pour faciliter la prise de la composition selon l'invention, le véhicule lipidique anhydre et hydrophobe peut comporter un ou plusieurs arômes et/ou des édulcorants classiquement utilisés dans les compositions pharmaceutiques.

Le véhicule lipidique anhydre et hydrophobe peut également comprendre un émulsifiant, comme par exemple les glycérostéarates et le cholestérol et/ou un fluidifiant, comme par exemple la diméthicone (polydiméthylsiloxane).

Tous les excipients additionnels utilisés doivent nécessairement être anhydres afin de préserver le potentiel osmotique du laxatif osmotique incorporé.

Les excipients de la composition sont tels qu'ils ne comprennent pas de dérivé cellulosique qui aurait pour effet indésirable de capter l'eau présente le long du tractus digestif entrainant une diminution de la pression osmotique du macrogol.

la présente invention, la composition pharmaceutique orale est constituée de :
- entre 30 et 55% en poids de la composition pharmaceutique de macrogol anhydre ; le macrogol peut être micronisé, dans ce cas, la taille de particules se situe de préférence entre 75 et 150 µm ; et
- entre 45 et 70% en poids de la composition pharmaceutique d'un véhicule constitué d'un enrobage lipidique anhydre et hydrophobe à base de composés gras d'origine végétale ; plus particulièrement, cet enrobage lipidique anhydre et hydrophobe est constitué :
   *soit jusqu'à 100% en poids du véhicule lorsqu'il s'agit de beurre de karité et/ou de beurre de cacao ; ou
   *soit de 12 à 25%, de préférence de 15 à 25%, en poids du véhicule d'une cire végétale ; et, selon qu'il s'agit de beurre végétal ou de cire végétale, de 25 à 88%, de préférence de 25 à 85%, en poids du véhicule d'une huile végétale permettant d'ajuster le point de fusion dudit véhicule entre 36 et 38°C ;
- entre 0 et 5% en poids de la composition pharmaceutique d'un excipient anhydre choisi parmi les fluidifiants, les agents améliorant l'homogénéité de la composition, les arômes, les édulcorants et/ou les émulsifiants à l'exclusion des dérivés cellulosiques.

La Demanderesse a pu mettre en évidence que, lorsque la composition pharmaceutique orale selon l'invention est administrée, le véhicule lipidique anhydre et hydrophobe isole et protège le macrogol de l'humidité du tractus digestif; le macrogol conserve ainsi jusqu'au colon sa pression osmotique proche de 100% au départ, d'où la réduction de posologie. Il est rappelé que le colon est le lieu communément reconnu de l'efficacité des laxatifs osmotiques.

Le ratio enrobage lipidique anhydre et hydrophobe sur actif est adapté pour résister au temps de vidage gastrique afin d'obtenir un délitement optimal et la libération du macrogol au niveau du colon, sans que le macrogol ne soit en contact direct avec l'humidité du tractus digestif qui aurait pour effet d'abaisser sa pression osmotique avant d'atteindre le colon.

La présente invention se rapporte avantageusement à la composition de l'invention pour son comme laxatif, en particulier pour le traitement de la constipation, de préférence chez l'adulte ; grâce à cette formulation optimisée, cette utilisation est telle que le macrogol est administré en une quantité représentant une posologie quotidienne réduite, comprise entre 5 à 15 g par jour pour un adulte à la différence des médicaments existants à base de macrogol dont la posologie oscille entre 10 et 30 g par jour.

Selon un mode de réalisation particulier, le véhicule est composé d'une cire végétale additionnée d'une huile végétale ; la composition pharmaceutique orale

| | |
|---|---|
| selon l'invention est alors constituée de : | |
| *Principe actif:* macrogol | de 45,00 à 55,00 g |
| *Excipient :* | |
| Cire végétale | de 6,00 à 11,00 g, de préférence de 9,00 à 11,00 g |
| Huile végétale | de 32,00 à 37,00 g |
| Optionnellement, agent améliorant l'homogénéité | de 1,80 à 1,90 g |
| Autres excipients tels que fluidifiant, émulsifiant, arômes et/ou édulcorants | de 1,60 à 1,80g |

Selon un mode de réalisation plus spécifique, la composition pharmaceutique orale selon l'invention est constituée de :

| | |
|---|---|
| *Principe actif:* macrogol | de 45,00 à 55,00 g |
| *Excipient :* | |
| Cire de carnauba | de 6,00 à 11,00 g, de préférence de 9,00 à 11,00 g |
| Huile de maïs | de 32,00 à 37,00 g |
| Optionnellement, du glycéryl stéarate | de 1,80 à 1,90 g |
| Autres excipients tels que fluidifiant, émulsifiant, arômes et/ou édulcorants | de 1,60 à 1,80g |

Le macrogol est un macrogol micronisé ou non, de type 4000 ou 3350.

Ce type de composition est préparé en mélangeant dans un premier temps la cire végétale et l'huile végétale dans un bain chauffant à environ 90°C ; le mélange est ensuite refroidi à environ 75°C et le glycéryl stéarate est éventuellement ajouté ; sont ensuite ajoutés les autres excipients ; le tout est agité jusqu'à l'obtention d'un mélange homogène ; le mélange est refroidi à environ 45°C et le principe actif est ajouté sous mélange.

Selon un autre mode de réalisation particulier, le véhicule est composé de beurre végétal et, éventuellement d'huile végétale ; la composition pharmaceutique orale selon l'invention est alors constituée de :
*Principe actif :* macrogol
   de 35,00 à 50,00 g, de préférence, de 35,00 à 45,00 g
*Excipients :*
   Beurre végétal (de karité et/ou de cacao) de 55,00 à 65,00 g

Autres excipients tels que fluidifiant, émulsifiant, arômes et/ou édulcorants
de 2,00 à 5,00 g, de préférence, de 2,00 à 4,00 g

Selon un autre mode de réalisation, la composition pharmaceutique orale selon l'invention est constituée de :

| | |
|---|---|
| *Principe actif* : macrogol micronisé | de 35,00 à 45,00 g |
| *Excipients :* | |
| Beurre végétal (de karité et/ou de cacao) | de 30,00 à 50,00 g |
| Huile végétale | de 15,00 à 25,00 g |

Autres excipients tels que fluidifiant, émulsifiant, arômes et/ou édulcorants de 2,00 à 5,00 g.

Selon encore un autre mode de réalisation, la composition pharmaceutique orale selon l'invention est constituée de :

| | |
|---|---|
| *Principe actif:* macrogol | de 35,00 à 45,00 g |
| *Excipients :* | |
| Beurre de karité | de 30,00 à 50,00 g |
| Beurre de cacao | de 3,00 à 10,00 g |
| Huile de mais | de 15,00 à 25,00 g |

Autres excipients tels que fluidifiant, émulsifiant, arômes et/ou édulcorants
de 2,00 à 5,00 g, de préférence, de 2,00 à 4,00 g

Le macrogol est un macrogol micronisé ou non, de type 4000 ou 3350.

Ce type de compositions est préparé par fonte du ou des beurres végétaux à une température comprise entre 45 et 50°C, ensuite incorporation de l'huile, puis ajout et mélange de l'actif micronisé et des éventuels autres excipients.

La présente description se rapporte encore à l'utilisation d'un véhicule anhydre et hydrophobe ayant un point de fusion compris entre 36 et 38°C constitué de composés gras d'origine végétale ayant un point de fusion compris entre 36 et 38°C tels que définis précédemment et optionnellement, jusqu'à 5% en poids d'un excipient choisi parmi des fluidifiants, des agents améliorant l'homogénéité, des émulsifiants, arômes et/ou édulcorants ; pour préserver la pression osmotique du macrogol jusqu'au colon. Par « préserver la pression osmotique du macrogol », on entend maintenir le macrogol dans un état anhydre (teneur en eau inférieure à 0,5%) jusqu'à ce qu'il ait atteint le colon, ce but est obtenu grâce au véhicule lipidique anhydre et hydrophobe qui empêche que le macrogol soit en contact avec l'humidité du tractus digestif qui abaisse sa pression osmotique, d'où la réduction de la posologie du macrogol dans la composition selon l'invention.

### EXEMPLES

### Exemples de compositions selon l'invention

| *Composition 1A* | |
|---|---|
| *Principe actif:* macrogol 4000 micronisé | 50,00 g |
| *Excipient :* | |
| Cire de carnauba | 9,65g |
| Huile de maïs | 36,70g |
| Glyceryl stéarate | 1,87g |
| Autres excipients | 1,78g |
| (Cholestérol, arome, aspartam) | |
| | |

| *Composition 1B* | |
|---|---|
| *Principe actif:* macrogol 4000 micronisé | 50,00 g |
| *Excipient :* | |
| Cire de carnauba | 6,20g |
| Huile de maïs | 38,70g |
| Glyceryl stéarate | 1,87g |
| Autres excipients | 3,23g |
| (Cholestérol, arome, aspartam) | |
| | |

| *Composition 2A* | |
|---|---|
| *Principe actif :* macrogol 3350 | 55,00 g |
| *Excipients :* | |
| Cire de carnauba | 9,35 g |
| Huile de tournesol | 33,87 g |
| Autres excipients | 1,78g |
| (Arôme, émulsifiant, édulcorant) | |
| | |

| *Composition 3A* | |
|---|---|
| *Principe actif :* macrogol 4000 micronisé | 35,00 g |
| *Excipients :* | |
| Beurre de karité | 62,00 g |
| Autres excipients | 3,00 g |
| (Arôme, émulsifiant, édulcorant, fluidifiant) | |
| | |

| *Composition 3B* | |
|---|---|
| *Principe actif :* macrogol 4000 micronisé | 40,00 g |
| *Excipients :* | |
| Beurre de karité | 35,00 g |
| Beurre de cacao | 5,00 g |
| Huile de mais | 17,00 g |
| Autres excipients | 3,00 g |
| (Arôme, émulsifiant, édulcorant, fluidifiant) | |

## Revendications

1. Composition pharmaceutique orale ayant pour but le maintien de la pression osmotique du macrogol lors de son administration orale et la réduction de la posologie journalière de ce laxatif, constituée de :
- entre 30 et 55% en poids de ladite composition pharmaceutique de macrogol anhydre ; et
- entre 45 et 70% en poids de la composition pharmaceutique d'un véhicule constitué d'un enrobage lipidique anhydre et hydrophobe à base de composés gras d'origine végétale ayant un point de fusion compris entre 36 et 38°C ;
- entre 0 et 5% en poids de la composition pharmaceutique d'un excipient anhydre choisi parmi des fluidifiants, des agents améliorant l'homogénéité, émulsifiants, arômes et/ou édulcorants à l'exclusion des dérivés cellulosiques.

2. Composition pharmaceutique orale selon la revendication 1, **caractérisé en ce que** ledit enrobage lipidique anhydre est constitué :
*soit jusqu'à 100% en poids du véhicule de beurre de karité et/ou de beurre de cacao ; ou
*soit de 12 à 25%, de préférence de 15 à 25%, en poids du véhicule d'une cire végétale ; et, selon qu'il s'agit de beurre végétal ou de cire végétale, de 25 à 88%, de préférence de 25 à 85%, en poids du véhicule d'une huile végétale permettant d'ajuster le point de fusion dudit véhicule entre 36 et 38°C.

3. Composition pharmaceutique orale selon la revendication 1 ou la revendication 2, **caractérisée en ce que** ledit laxatif osmotique est micronisé ou non micronisé.

4. Composition pharmaceutique orale selon la revendication 2 , **caractérisée en ce que** la cire végétale est la cire de carnauba, la cire de candelilla, la cire de son de riz, ou une autre cire végétale pharmaceutiquement acceptable.

5. Composition pharmaceutique orale selon l'une quelconque des revendications 2 ou 4, **caractérisée en ce que** l'huile végétale est choisie parmi l'huile de tournesol, l'huile de colza, l'huile de maïs, l'huile de lin ou leur mélange.

6. Composition pharmaceutique orale selon l'une quelconque des revendications précédentes, **caractérisée en ce qu'**elle est constituée de :
| | |
|---|---|
| *Principe actif:* macrogol | de 45,00 à 55,00 g |
| *Excipient :* | |
| Cire végétale | de 6,00 à 11,00 g |
| Huile végétale | de 32,00 à 37,00 g |
| Agent améliorant l'homogénéité | de 1,80 à 1,90 g |
| Autres excipients tels que fluidifiant, émulsifiant, arômes et/ou édulcorants | de 1,60 à 1,80 g. |

7. Composition pharmaceutique orale selon l'une quelconque des revendications précédentes, **caractérisée en ce qu'**elle est constituée de :
| | |
|---|---|
| *Principe actif:* macrogol | de 45,00 à 55,00 g |
| *Excipient :* | |
| Cire de carnauba | de 6,00 à 11,00 g |
| Huile de maïs | de 32,00 à 37,00 g |
| Optionnellement du glycéryl stéarate | de 1,80 à 1,90 g |
| Autres excipients tels que fluidifiant, émulsifiant, arômes et/ou édulcorants | de 1,60 à 1,80 g. |

8. Composition pharmaceutique orale selon l'une quelconque des revendications 1 à 3 ou 5, **caractérisée en ce qu'**elle est constituée de :
| | |
|---|---|
| *Principe actif:* macrogol micronisé | de 35,00 à 45,00 g |
| *Excipients :* | |
| Beurre végétal (de karité et/ou de cacao) | de 30,00 à 50,00 g |
| Huile végétale | de 15,00 à 25,00 g |
| Autres excipients tels que fluidifiant, émulsifiant, arômes et/ou édulcorants | de 2,00 à 5,00 g. |

9. Composition pharmaceutique orale selon la revendication 8, **caractérisée en ce qu'**elle est constituée de :
| | |
|---|---|
| *Principe actif:* macrogol micronisé | de 35,00 à 45,00 g |
| *Excipients :* | |
| Beurre de karité | de 30,00 à 50,00 g |
| Beurre de cacao | de 3,00 à 10,00 g |
| Huile de mais | de 15,00 à 25,00 g |
| Autres excipients tels que fluidifiant, émulsifiant, arômes et/ou édulcorants | de 2,00 à 5,00 g. |

10. Composition pharmaceutique orale selon l'une quelconque des revendications précédentes pour son utilisation comme laxatif pour le traitement de la constipation chez l'adulte **caractérisé en ce que** ladite composition est administrée en une quantité représentant un apport de macrogol réduit à 5 à 15 g par jour grâce à une protection de sa pression osmotique jusqu'au colon ; site de son action thérapeutique.

11. Composition pharmaceutique orale selon l'une quelconque des revendications 1 à 9 pour son utilisation selon la revendication 10, **caractérisée en ce que** son administration réduit les effets secondaires indésirables choisis parmi les crampes abdominales, les ballonnements et les flatulences.

## Patentansprüche

1. Orale pharmazeutische Zusammensetzung, die zum Ziel das Aufrechterhalten des osmotischen Drucks des Macrogols bei seiner oralen Verabreichung und die Verringerung der Tagesdosis dieses Abführmittels aufweist, bestehend aus:
- zwischen 30 und 55 Gewichts-% der pharmazeutischen Zusammensetzung aus wasserfreiem Macrogol; und
- zwischen 45 und 70 Gewichts-% der pharmazeutischen Zusammensetzung aus einem Träger, bestehend aus einer wasserfreien und hydrophoben Lipidbeschichtung auf Basis von Fettverbindungen pflanzlichen Ursprungs mit einem Schmelzpunkt im Bereich zwischen 36 und 38 °C;
- zwischen 0 und 5 Gewichts-% der pharmazeutischen Zusammensetzung aus einem wasserfreien Hilfsstoff, ausgewählt aus Verflüssigungsmitteln, die Homogenität verbessernde Mittel, Emulgatoren, Aromen und/oder Süßungsmitteln mit Ausnahme von Cellulosederivaten.

2. Orale pharmazeutische Zusammensetzung nach Anspruch 1, **dadurch gekennzeichnet, dass** die wasserfreie Lipidbeschichtung besteht aus:
*entweder bis zu 100 Gewichts-% des Trägers aus Sheabutter und/oder aus Kakaobutter; oder
*oder von 12 bis 25 Gewichts-%, vorzugsweise von 15 bis 25 Gewichts-% des Trägers aus einem pflanzlichen Wachs; und, je nachdem, ob es sich um pflanzliche Butter oder um pflanzliches Wachs handelt, von 25 bis 88 Gewichts-%, vorzugsweise von 25 bis 85 Gewichts-% des Trägers aus einem pflanzlichen Öl, das gestattet, den Schmelzpunkt des Trägers zwischen 36 und 38 °C einzustellen.

3. Orale pharmazeutische Zusammensetzung nach Anspruch 1 oder Anspruch 2, **dadurch gekennzeichnet, dass** das osmotische Abführmittel mikronisiert oder nicht mikronisiert ist.

4. Orale pharmazeutische Zusammensetzung nach Anspruch 2, **dadurch gekennzeichnet, dass** das pflanzliche Wachs Carnaubawachs, Candelillawachs, Reiskleiewachs oder ein anderes pharmazeutisch annehmbares pflanzliches Wachs ist.

5. Orale pharmazeutische Zusammensetzung nach einem der Ansprüche 2 oder 4, **dadurch gekennzeichnet, dass** das pflanzliche Öl ausgewählt ist aus Sonnenblumenöl, Rapsöl, Maisöl, Leinöl oder deren Gemisch.

6. Orale pharmazeutische Zusammensetzung nach einem der vorstehenden Ansprüche, **dadurch gekennzeichnet, dass** sie besteht aus:
| | |
|---|---|
| *Wirkstoff:* Macrogol | von 45,00 bis 55,00 g |
| *Hilfsstoff:* | |
| pflanzliches Wachs | von 6,00 bis 11,00 g |
| pflanzliches Öl | von 32,00 bis 37,00 g |
| die Homogenität verbesserndes Mittel | von 1,80 bis 1,90 g |
| andere Hilfsstoffe wie Verflüssigungsmittel, Emulgator, Aromen und/oder Süßungsmittel | von 1,60 bis 1,80 g. |

7. Orale pharmazeutische Zusammensetzung nach einem der vorstehenden Ansprüche, **dadurch gekennzeichnet, dass** sie besteht aus:
| | |
|---|---|
| *Wirkstoff:* Macrogol | von 45,00 bis 55,00 g |
| *Hilfsstoff:* | |
| Carnaubawachs | von 6,00 bis 11,00 g |
| Maisöl | von 32,00 bis 37,00 g |
| wahlweise Glycerylstearat | von 1,80 bis 1,90 g |
| andere Hilfsstoffe wie Verflüssigungsmittel, Emulgator, Aromen und/oder Süßungsmittel | von 1,60 bis 1,80 g. |

8. Orale pharmazeutische Zusammensetzung nach einem der Ansprüche 1 bis 3 oder 5, **dadurch gekennzeichnet, dass** sie besteht aus:
| | |
|---|---|
| *Wirkstoff:* mikronisiertes Macrogol | von 35,00 bis 45,00 g |
| *Hilfsstoffe:* | |
| pflanzliche Butter (von Shea und/oder von Kakao) | von 30,00 bis 50,00 g |
| pflanzliches Öl | von 15,00 bis 25,00 g |
| andere Hilfsstoffe wie Verflüssigungsmittel, Emulgator, Aromen und/oder Süßungsmittel | von 2,00 bis 5,00 g. |

9. Orale pharmazeutische Zusammensetzung nach Anspruch 8, **dadurch gekennzeichnet, dass** sie besteht aus:
| | |
|---|---|
| *Wirkstoff:* mikronisiertes Macrogol | von 35,00 bis 45,00 g |
| *Hilfsstoffe:* | |
| Sheabutter | von 30,00 bis 50,00 g |
| Kakaobutter | von 3,00 bis 10,00 g |
| Maisöl | von 15,00 bis 25,00 g |
| andere Hilfsstoffe wie Verflüssigungsmittel, Emulgator, Aromen und/oder Süßungsmittel | von 2,00 bis 5,00 g. |

10. Orale pharmazeutische Zusammensetzung nach einem der vorstehenden Ansprüche zu deren Verwendung als Abführmittel zur Behandlung von Verstopfung bei Erwachsenen, **dadurch gekennzeichnet, dass** die Zusammensetzung eine Menge, die eine auf 5 bis 15 g pro Tag verringerte Zufuhr an Macrogol darstellt, Dank eines Schutzes ihres osmotischen Drucks bis zum Kolon; Ort ihrer therapeutischen Wirkung; verabreicht wird.

11. Orale pharmazeutische Zusammensetzung nach einem der Ansprüche 1 bis 9 zu deren Verwendung nach Anspruch 10, **dadurch gekennzeichnet, dass** ihre Verabreichung die unerwünschten Nebenwirkungen, ausgewählt aus Unterleibskrämpfen, Blähungen und Flatulenz, verringert.

## Claims

1. Oral pharmaceutical composition having the aim of maintaining osmotic pressure of macrogol during its oral administration and of reducing the daily dosage of this laxative, constituted of:
- between 30 and 55% by weight of said pharmaceutical composition of anhydrous macrogol; and
- between 45 and 70% by weight of the pharmaceutical composition of a vehicle constituted of an anhydrous and hydrophobic lipid coating based on fatty compounds of plant origin having a melting point comprised between 36 and 38°C;
- between 0 and 5% by weight of the pharmaceutical composition of an anhydrous excipient chosen from among thinners, agents improving homogeneity, emulsifiers, flavourings and/or sweeteners, excluding cellulosic derivatives.

2. Oral pharmaceutical composition according to claim 1, **characterised in that** said anhydrous lipid coating constituted of:
* either up to 100% by weight of the shea butter and/or cocoa butter vehicle; or
* from 12 to 25%, preferably from 15 to 25%, by weight of the vehicle of a plant wax;
and, depending to whether it is plant butter or plant wax, from 25 to 88%, preferably from 25 to 85%, by weight of the vehicle of a plant oil making it possible to adjust the melting point of said vehicle between 36 and 38°C.

3. Oral pharmaceutical composition according to claim 1 or claim 2, **characterised in that** said osmotic laxative is micronised or non-micronised.

4. Oral pharmaceutical composition according to claim 2, **characterised in that** the plant wax is carnauba wax, candelilla wax, rice bran wax, or another pharmaceutically acceptable plant wax.

5. Oral pharmaceutical composition according to any one of claims 2 or 4, **characterised in that** the plant oil is chosen from among sunflower oil, rape oil, corn oil, linseed oil or the mixture thereof.

6. Oral pharmaceutical composition according to any one of the preceding claims, **characterised in that** it is constituted of:
| | |
|---|---|
| *Active ingredient:* macrogol | from 45.00 to 55.00g |
| *Excipient:* | |
| Plant wax | from 6.00 to 11.00g |
| Plant oil | from 32.00 to 37.00g |
| Agent improving homogeneity | from 1.80 to 1.90g |
| Other excipients such as thinner, emulsifier, flavourings and/or sweeteners | from 1.60 to 1.80g. |

7. Oral pharmaceutical composition according to any one of the preceding claims, **characterised in that** it is constituted of:
| | |
|---|---|
| *Active ingredient:* macrogol | from 45.00 to 55.00g |
| *Excipient:* | |
| Carnauba wax | from 6.00 to 11.00g |
| Corn oil | from 32.00 to 37.00g |
| Optionally glycerol stearate | from 1.80 to 1.90g |
| Other excipients such as thinner, emulsifier, flavourings and/or sweeteners | from 1.60 to 1.80g. |

8. Oral pharmaceutical composition according to any one of claims 1 to 3 or 5, **characterised in that** it is constituted of:
| | |
|---|---|
| *Active ingredient:* micronised macrogol | from 35.00 to 45.00g |
| *Excipients:* | |
| Plant butter (shea and/or cocoa) | from 30.00 to 50.00g |
| Plant oil | from 15.00 to 25.00g |
| Other excipients such as thinner, emulsifier, flavourings and/or sweeteners | from 2.00 to 5.00g. |

9. Oral pharmaceutical composition according to claim 8, **characterised in that** it is constituted of:
| | |
|---|---|
| *Active ingredient:* micronised macrogol | from 35.00 to 45.00g |
| *Excipients:* | |
| Shea butter | from 30.00 to 50.00g |
| Cocoa butter | from 3.00 to 10.00g |
| Corn oil | from 15.00 to 25.00g |
| Other excipients such as thinner, emulsifier, flavourings and/or sweeteners | from 2.00 to 5.00g. |

10. Oral pharmaceutical composition according to any one of the preceding claims for its use as laxative for the treatment of constipation in adults, **characterised in that** said composition is administered in a quantity representing an input of macrogol reduced to 5 to 15g per day thanks to a protection of its osmotic pressure up to the colon; site of its therapeutic action.

11. Oral pharmaceutical composition according to any one of claims 1 to 9, for its use according to claim 10, **characterised in that** its administration reduces the undesirable side effects chosen from among abdominal cramps, bloating and flatulence.
